# EUROPÄISCHE PATENTSCHRIFT

(11) **EP 1 256 380 B1**
(45) Veröffentlichungstag und Bekanntmachung des Hinweises auf die Patenterteilung: **25.08.2004**
(21) Anmeldenummer: 01111312.3
(22) Anmeldetag: 09.05.2001
(51) Int. Cl.: B01J 20/32, A61M 1/36

(54) **Adsorbentien zur Perfusion von Blut und deren Herstellungsverfahren**
Adsorbents for blood perfusion and their preparation method
Adsorbants pour perfusion sanguine et leur méthode de préparation

(43) Veröffentlichungstag der Anmeldung: 13.11.2002
(73) Patentinhaber: Thies, Karsten, Dr., 64668 Rimbach (DE)
(72) Erfinder: Thies, Karsten, Dr., 64668 Rimbach (DE)

(56) Entgegenhaltungen:
- EP-A- 0 143 369
- EP-A- 0 464 872
- WO-A-90/09238
- DE-A- 3 617 672
- DE-A- 4 018 778
- US-A- 4 298 500

## Beschreibung

Die Erfindung betrifft blutverträgliche Adsorbentien, insbesondere zur Entfernung von atherogenen Lipoproteinen, vor allem Low Density Lipoprotein (LDL), aus Blut oder Plasma mittels Perfusion. Des weiteren beinhaltet die Erfindung ein Verfahren zur Herstellung dieser Adsorbentien.

Im Rahmen extrakorporaler Blutbehandlungsverfahren ist die Säulenchromatographie mit Adsorbentien ein geeignetes Verfahren zur Elimination von Makromolekülen, wie z.B. Antikörpern, Lipopolysacchariden, arteriosklerosefördernden Lipoproteinen, β₂-Mikroglobulin u.a. sowie von kleinen, eiweißgebundenen Stoffen, wie beispielsweise Toxinen, Bilirubin u.a. Auch die selektive Entfernung von Zellpopulationen, wie z.B. die Isolierung von Blutstammzellen oder die gezielte Elimination anderer leukozytärer Subpopulationen, ist möglich. Dabei wird das Prinzip der Affinitätschromatographie oder der Adsorptionschromatographie angewandt, d.h. eine adsorbierende Oberfläche oder ein spezifischer Ligand binden die zu eliminierende Substanz oder Zelle.

Derartige Blutreinigungsverfahren werden derzeit meistens nach vorheriger Abtrennung der Blutzellen mittels Zellseparator oder Filter durchgeführt, so dass nur das zu reinigende Blutplasma durch die Adsorptionssäule geleitet wird. Dies ermöglicht es, auch solche Adsorbentien einzusetzen, die Blutzellen binden oder aktivieren würden und deshalb zur Blutperfusion nicht geeignet sind.

Neuerdings wird angestrebt, Verfahren, die bislang nur mit Plasma möglich waren, vermehrt als direkte Hämoperfusion in kürzerer Behandlungszeit mit weniger apparativem Aufwand und mit weniger Personal durchzuführen. Damit können die Behandlungskosten gesenkt werden und das Verfahren mehr Patienten zugänglich gemacht werden.

In DE 3932971 A1 werden dazu poröse, sphärische Homo-, Co- bzw. Terpolymere von Vinylverbindungen, wie beispielsweise Acrylsäure, als Trägermaterialien verwendet ( organische Hartgele). Diese werden, teilweise über organische Brückenglieder (Spacer), mit kovalent gebundenen organischen Liganden spezifischer Wirkungsrichtung versehen. Mit Polyacrylsäure als organischem Liganden sind sie für die Eliminationsbehandlung von Lipoproteinen geringer Dichte (LDL) direkt aus Blut geeignet.
Organische Hartgele können jedoch für diesen Anwendungszweck nicht als optimal gelten. Zwar weisen sie eine ausreichende Druckstabilität für die Blutperfusion auf, es wurde aber festgestellt, dass während einer Behandlung eine relativ große Zahl von Mikroteilchen des Adsorbers in die Blutbahn gelangen kann. Auch ist das Risiko einer Allergieauslösung durch das synthetisch hergestellte Trägermaterial grundsätzlich gegeben.
Ein Nachteil bezüglich der Elimination von LDL ist eine im Vergleich zu anorganischen Trägermaterialien geringere Adsorptionskapazität.
Obwohl bezüglich dieser Merkmale anorganische poröse Trägermaterialien wie Glas oder Kieselgel vorteilhaft sind, ist wegen ihrer Blutunverträglichkeit eine vergleichbare klinische Anwendung der Hämoperfusion bisher nicht bekannt geworden.
Nachteilig gegenüber organischen Trägermaterialien ist die bekannte unspezifische Adsorption, insbesondere von Proteinen, durch anionische SiO⁻ -Gruppen auf ihrer Oberfläche.
Aus Journal Chromatogr. Sci. 14, 316 (1976) und aus Journal Chromatogr. 125, 103 (1976) ist die Silanisierung von porösem Glas und Kieselgel mit 3-Glycidoxypropyltrimethoxysilan und Hydrolyse der Epoxidfunktion zur Diolgruppierung bekannt. Damit werden freie Silanolgruppen (SiO⁻) blockiert und eine hydrophile Oberfläche erzeugt. Zur Herstellung von Gelfiltrationsmedien mit geringerer unspezifischer Adsorption hat sich dieses Verfahren bewährt.
Aus US-A-4 298 500 ist weiterhin bekannt, dass sich Mischsilanisierungen der genannten anorganischen Trägermaterialien mit einem tertiären Aminosilan und mit 3-Glycidoxypropyltrimethoxysilan oder einem Trialkoxy-Diolsilan zur Herstellung von Ionenaustauschern mit reduzierter Ladungsdichte eignen. Gegenüber herkömmlichen Ionenaustauschern weisen sie eine bessere Eluierbarkeit und geringere irreversible Bindung von Biomolekülen auf. Die Möglichkeit der Anbindung eines Affinitätsliganden, wie z.B. von Polyacrylsäure, war dagegen nicht vorgesehen und wegen des Fehlens primärer Aminogruppen auch nicht gegeben. Die genannten Ionenaustauscher waren zudem nicht für die extrakorporale Perfusion vorgesehen, so dass keine Daten zu ihrer Blutverträglichkeit vorliegen.
Bei der Perfusion von Blut können beispielsweise Gerinnungsfaktoren unspezifisch adsorbiert und trotz Antikoagulation aktiviert werden. Bereits geringe Mengen von Fibrin induzieren eine Adhäsion und Aggregation von Thrombozyten, die wiederum gerinnungsaktive und die Zelladhärenz fördernde Substanzen freisetzen. Dadurch erfolgt zusätzlich eine Retention von Leukozyten und Gerinnselbildung, die auch Erythrozyten einschließt. Diese Mechanismen führen um so schneller zu einer vollständigen Verlegung der interpartikulären Zwischenräume durch Blutgerinnsel, je kleiner die Trägerpartikel und damit die Zwischenräume sind.
Neben der Gerinnung ist insbesondere das Ausmaß der Zelladhäsion für die Blutverträglichkeit von Bedeutung, da anhaftende Zellen ebenfalls zur Erhöhung des Strömungswiderstandes führen können. Eine unvollständige Elusion anhaftender Erythrozyten nach einer Blutperfusion ist beispielsweise ein Hinweis auf mangelnde Blutverträglichkeit.
Im Stand der Technik wurde verschiedentlich versucht, durch Bindung einer gerinnungshemmenden, antithrombogenen Substanz an Trägermaterialien die Blutverträglichkeit zu verbessern.
Beispielsweise betrifft die Patentschrift DE 2532883 C2 ein Hämoperfusionsverfahren mittels eines porösen Glasgranulates, welches nach Behandlung mit einem Aminosilan mit Glutaraldehyd umgesetzt wurde, so dass an dieses die kovalente Bindung von Antigenen oder Antikörpern erfolgen konnte. Diese Glasderivate erlaubten eine nebenwirkungsfreie, regenerierbare und mehrfache Verwendung zur selektiven Gewinnung eines Reaktionspartners einer Immunreaktion aus dem strömenden Blut von lebenden Organismen. Das Verfahren war aber nur möglich, wenn die Thrombogenität der Oberfläche durch zusätzliche kovalente Bindung relativ großer Heparinmengen bei gleichzeitiger intravenöser Verabreichung von Heparin ausgeschaltet wurde. Unterblieb der Schritt, konnte selbst exzessive Antikoagulation des Blutes eine rasche, vollständige Verlegung der Adsorbersäule mit Thromben nicht verhindern.

DE 36 17 672 C2 beschreibt organische und anorganische Reagentien zum Zwecke der LDL-Eliminationsbehandlung aus Körperflüssigkeiten wie Blut, Plasma oder Serum, wobei u.a. an eine Silica-Trägermatrix nach Silanisierung mit einer eine Epoxid- Funktionalität aufweisenden Verbindung über einen Aminogruppenhaltigen Spacer eine Polycarbonsäure, wie Heparin bzw. alternativ eine Polyacrylsäure gebunden ist. Obwohl die Möglichkeit einer Adsorption aus Blut in Betracht gezogen wird, ist weder ausgeführt, welches der genannten Trägermaterialien dafür verwendet werden soll, noch ist ein Beispiel eines Blutversuches offenbart. Vielmehr erfolgten alle Anwendungsbeispiele mit Plasma oder Serum. Die relativ aufwendige Technik zur Herstellung führt zudem zu einer ungünstigen Kosten-Nutzen-Relation.
Bei dem in EP 0143 369 A2 beschriebenen Adsorbens findet zum Zwecke der extrakorporalen Elimination von LDL neben organischen Trägern auch CPG, dass mit Amino-terminierter Polyacrylsäure beschichtet ist, als Trägermaterial Verwendung. Auch hier erfolgt zunächst eine Silanisierung mit einem eine Epoxygruppe aufweisenden Silan, an das, in diesem Fall über das terminale Amin, die Polyacrylsäure kovalent gebunden ist. Bei der Beschichtung ist es explizit beabsichtigt, dass neben der durch die Polyacrylsäure bedingten negativen Ladung auf der Oberfläche auch freie Silanolgruppen des CPG vorliegen. Im Hinblick auf eine mögliche Thrombusbildung bei der Verwendung insbesondere von Blut erfolgt lediglich bei den beschichteten organischen Trägem, die keine freien Silanolgruppen, sondern lediglich die Polyacrylsäure auf der Trägeroberfläche aufweisen, der Hinweis, dass diese sich durch eine geringe Neigung zur Thrombusbildung bei der Verwendung von Blut auszeichnen. Bei Verwendung der CPG-Trägermatrix, bei der jedoch freie Silanolgruppen auf der Trägeroberfläche vorhanden sind, sollte daher keine Blutkompatibilität gegeben sein.
In DE 40 18 778 A1 erfolgt eine Adsorption verschiedener Lipoproteine aus Blut, Plasma oder Serum an ein Adsorbens, bestehend aus porösen Glaskörpern als Trägermaterial mit Liganden, welche mindestens eine Ethergruppierung mit einer endständigen α,β-Diolgruppe enthaltende Alkylreste mit 4 bis 12 C-Atomen aufweisen und keine freien Silanolgruppen mehr enthalten sollen. Der Patentschrift ist zu entnehmen, dass im bevorzugten Fall lediglich spezielle Molekülgruppen dieser Definition bzw. ihre Kombination (z.B. 1,2-Dihydroxypropyl-3-oxypropyl-, 1-Hydroxyethyl-3-oxypropyl- und 1,1-Dimethoxyethyl-2-oxypropylreste im Verhältnis 1 : 1,3 : 0,8) zufriedenstellende LDL -Bindung bewirken. Ein Polyanion wird jedoch nicht verwendet. Zur Anwendbarkeit mit Blut wird lediglich festgestellt, dass keine Hämolyse auftritt.

Bei allen bisher beschriebenen Adsorbentien verlief die Perfusion von Blut bisher nur entweder unter Verwendung organischer Trägermaterialien oder unter Einsatz von Heparin als Beschichtungsmaterial bzw. als Antikoagulans im Blut.

Deshalb liegt der vorliegenden Erfindung die Aufgabe zu Grunde, ein blutkompatibles Adsorbens zur Hämoperfusion bereitzustellen, das die oben dargestellten Vorteile von porösen, anorganischen siliciumhaltigen Trägermaterialien verbindet mit der Fähigkeit, ohne Verwendung von Heparin oder sonstigen aufwendigen Verfahren die Blutunverträglichkeit auszuschalten.

Die Aufgabe wird erfindungsgemäß gelöst durch die Bereitstellung eines Adsorbens zur Perfusion von Blut oder Plasma, bestehend aus einem festen, siliciumhaltigen, anorganischen, porösen Trägermaterial, an das zumindest ein hydrophiles und ein kupplungsfähiges Organosilan gebunden sind, wobei das hydrophile Organosilan ein Diolsilan und das kupplungsfähige Organosilan ein Aminosilan ist und
a) ein Polyanion kovalent an das Aminosilan gebunden ist, wobei das Polyanion ein Acrylpolymer ist,
b) keine freien Silanolgruppen auf der Trägeroberfläche mehr vorhanden sind und
c) der Anteil des Aminosilans relativ zum Diolsilan 0,1-20% beträgt.

Das Adsorbens weist keine Zellretention auf.

Überraschenderweise hat sich gezeigt, dass auch ohne Verwendung von Heparin, d. h. mit ausschließlicher Citratantikoagulation, eine ausgezeichnete Blutkompatibilität erzielt wird, wenn einerseits die Silanolgruppen so durch mindestens ein Diolsilan und ein Aminosilan bedeckt werden, dass keine freien Silanolgruppen mehr nachweisbar sind und andererseits an das Aminosilan über eine vorgegebene Zahl von Bindungsstellen ein Acrylpolymer als monomolekulare Schicht gebunden wird.

In einer bevorzugten Ausführungsform wird das Diolsilan durch Hydrolyse in situ aus einem Epoxysilan, besonders bevorzugt aus 3-Glycidoxypropyltrimethoxysilan, erzeugt, so dass es sich bei dem Diolsilan besonders bevorzugt um 1,2-Dihydroxypropyl-3-oxypropylsilan handelt. Als Aminosilan hat sich insbesondere 3-Aminopropyltriethoxysilan als besonders vorteilhaft herausgestellt. Verbindungen mit einem hohen hydrophoben Anteil sollten zur Bedeckung der Trägeroberfläche vermieden werden, da sie u.a. durch Bindung von Erythrozyten die Blutverträglichkeit herabsetzen.

Das Aminosilan dient als Bindungsstelle zur kovalenten Bindung des Acrylpolymers und kann vorteilhafterweise im Silanisierungsschritt gleichzeitig mit dem Diolsilan aufgebracht werden. Dies ermöglicht es, die Zahl der Bindungsstellen auf dem Träger über die Konzentration des Aminosilans sehr leicht und genau vorzugeben.

Die Untersuchungen, die zum erfindungsgemäßen Adsorbens geführt haben, zeigten, dass die Adsorption insbesondere von Lipoproteinen geringer und sehr geringer Dichte (LDL bzw. VLDL) über das Acrylpolymer der Trägeroberfläche mit deutlich höherer Kapazität und Selektivität erfolgt, wenn diese jeweils über möglichst wenige Bindungsstellen, bevorzugt über eine 1-Punktbindung, auf der Trägeroberfläche fixiert ist. Daher liegt der Anteil des Aminosilans auf der Trägeroberfläche relativ zu dem Diolsilan zwischen 0,1 und 20%. Das so beschichtete Trägermaterial ergibt beim Erhitzen in Ninhydrinreagenz lediglich eine schwache Blaufärbung als Hinweis auf die erwünschte geringe Dichte reaktiver Aminogruppen.

Ist der Anteil des Aminosilans größer als 20%, so äußert sich dies in einer Abnahme der Kapazität und der Selektivität, vermutlich durch Fixierung der Polyacrylsäure an mehrere Bindungsstellen, einhergehend mit einer Reduktion ihrer freien Beweglichkeit.

Auch hat sich herausgestellt, dass ein hoher Aminogruppenanteil mit einer schlechteren Blutverträglichkeit und geringerer Stabilität des Adsorbens verbunden ist. Unterhalb einer kritischen Dichte von angebotenen Aminogruppen sinkt die Bindungskapazität wieder ab, da dann zu wenig Polyacrylsäure-Moleküle gebunden werden.
Die Abwesenheit freier Silanolgruppen auf der Trägeroberfläche nach der Silanisierung lässt sich bei Versetzen des getrockneten Trägermaterials mit einer 0,05%igen Lösung von Methylrot in Toluol durch das Ausbleiben eines aufgrund von Bindung und Metachromasie induzierten Farbumschlages von gelb zu leuchtend rot auf der Oberfläche sehr empfindlich nachweisen. Im Gegensatz zu der in EP 0143369 A2 beschriebenen Trägermatrix aus porösem Glas ist es bei dem vorliegenden erfindungsgemäßen Adsorbens erforderlich, dass keine freien Silanolgruppen auf der Trägeroberfläche mehr nachweisbar sind. Dadurch wird die Thrombogenität herabgesetzt und die Retention von Thrombozyten, nicht jedoch die Adsorption von Erythrozyten vollständig vermieden.

Im Gegensatz zu den in DE 4018778 A1 beschriebenen Adsorbentien bindet ein derart beschichtetes Trägermaterial keine Lipoproteine (siehe Tabelle 1, Beispiel 5).
Die Lipoproteinbindung erfolgt im Gegensatz zu den beiden zuvor genannten Patentschriften somit allein über das Polyanion, wie auch aus der vollständigen Eluierbarkeit mittels einer Lösung von Polyanionen erkennbar ist. Bei dem gebundenen Polyanion handelt es sich um ein Acrylpolymer, insbesondere um ein Polymer oder Copolymer der Acrylsäure, Methacrylsäure oder Hydroxyethylmethacrylsäure. Es weist vorzugsweise ein mittleres Molekulargewicht von 5x10³ D bis 5x10⁶ D, besonders bevorzugt von 5x10⁴ D bis 5x10⁵ D auf.

Für die Perfusion von Blut durch eine mit Trägermaterial gefüllte Säule müssen die Blutzellen die interpartikulären Zwischenräume ungehindert passieren können. Optimal wäre es daher, eine möglichst große Korngröße der Partikel, wie beispielsweise 0,5 mm, zu wählen. Dies ist aus chromatographischen Gründen jedoch nicht zu empfehlen, da die Diffusionsstrecken zur adsorbierenden Oberfläche im Partikelinneren dann zu lang sind, so daß eine ungünstige Bindungskinetik resultiert. Infolgedessen verläßt ein Teil der zu bindenden Substanz die Säule ungebunden, bevor die Bindungskapazität erschöpft ist. Ist der Partikeldurchmesser zu klein gewählt, so sind die interpartikulären Zwischenräume zu klein, um die Blutzellen passieren zu lassen.

Im Gegensatz zu sphärisch geformtem Trägermaterial unterliegen die interpartikulären Zwischenräume bei den völlig unregelmäßig geformten Glas- oder Kieselgelgranulaten größeren Schwankungen. Um eine ungehinderte Passage aller Blutzellen zu erlauben, sind in diesem Fall Partikel mit einer Korngröße von 50 bis 250 µm bevorzugt und von 100 bis 200 µm besonders bevorzugt.

Ein weiterer Gegenstand der vorliegenden Erfindung betrifft ein Verfahren zur Herstellung des oben beschriebenen Adsorbens, das folgende Schritte beinhaltet:
a) Bindung eines Aminosilans und eines Diolsilans an die Trägeroberfläche unter vollständiger Umsetzung der freien Silanolgruppen auf der Trägeroberfläche
b) Kupplung eines Acrylpolymers in einem pH-Wertbereich von 1,5 bis 8,5

Vorteilhafterweise erfolgt die Silanisierung unter sauren Bedingungen, d. h. in einem pH-Wertbereich von 2 bis 6, im Temperaturbereich von 70 bis 150°C bei einer Reaktionszeit von 0,5 bis 8 Stunden. Auf diese Weise wird eine vollständige Umsetzung der freien Silanolgruppen der Trägeroberfläche sichergestellt. Besonders vorteilhaft erfolgt die Silanisierung mit einer 0,5-10 %igen, bevorzugt mit einer 1-5 %igen wässrigen Lösung von 1,2- Dihydroxypropyl-3-oxypropylsilan und 0,01 bis 1%, bevorzugt 0,0125% bis 0,25% Aminopropyltriethoxysilan bei pH 3 bis 4, bevorzugt 3,5, für 5 Stunden in einem siedenden Wasserbad. Zur Verbesserung der Stabilität, insbesondere wenn ein Sterilisationsverfahren mit Hitze vorgesehen ist, ist es möglich, die Silanbeschichtung auch allein oder zusätzlich zu dem oben beschriebenen Verfahren bei 121 °C bis 150°C im Autoklaven durchzuführen. Wie im Stand der Technik bereits bekannt ist, kann eine auf die Silanbeschichtung folgende Trocknung bei höheren Temperaturen wie beispielsweise 80 bis 150°C ggf. im Vakuum erfolgen, um eine Ausbildung von -Si-O-Si- Brücken unter Abspaltung von Wasser und damit eine erhöhte Stabilität der Beschichtung zu bewirken.

Im Gegensatz zum bisherigen Stand der Technik wird in einer bevorzugten AusFührung der Erfindung die Epoxyverbindung, bevorzugt 3-Glycidoxypropyltrimethoxysilan, bereits vor der Kopplung auf die Trägeroberfläche hydrolisiert, so dass die Silanisierung entsprechend mit dem Diolsilan erfolgt. Dieses Vorgehen bietet zahlreiche Vorteile:
- Die Hydrolyse erfolgt in Lösung vollständiger und schneller als auf einer Oberfläche, wie z.B. der Trägeroberfläche.
- Die Gefahr der Silanablösung von der Trägeroberfläche durch die zur Epoxidspaltung eingesetzte Säure mit erneuter Exposition freier Silanolgruppen wird vermieden.
- Diolgruppen und Aminogruppen können in einem Reaktionsschritt aufgebracht werden, so dass der apparative und finanzielle Aufwand bei der Herstellung verringert wird.
- Das Verhältnis des Diolsilans zum Aminosilan ist leichter steuerbar und gut reproduzierbar.
- Die Silanisierung kann bei höherer Temperatur ohne Rücksicht auf unerwünschte Reaktionen der Epoxygruppe erfolgen.

Es hat sich zudem gezeigt, dass die Herstellung des Adsorbens gemäß dem beschriebenen erfindungsgemäßen Verfahren bezüglich der Blutkompatibilität gegenüber anderen Verfahren überlegen war.

Bei Schritt b) handelt es sich um eine Acrylpolymerkopplung. Diese wird in einem pH-Wertbereich von 1,5 bis 8,5, bevorzugt in einem pH-Wertbereich von 3,0 bis 5,5 durchgeführt. Bevorzugt wird als Acrylpolymer Polyacrylsäure verwendet. Es ist besonders bevorzugt, eine Polyacrylsäure mit einem mittleren Molekulargewicht von 5 x 10³ bis 5 x 10⁶ D, vorzugsweise 5 x 10⁴ bis 5 x 10⁵ D, einzusetzen.

Aus dem Stand der Technik ist ein Verfahren bekannt, bei dem Polyacrylsäureanhydrid direkt an Amino-derivatisiertes Kieselgel oder Glas gebunden und anschließend hydrolisiert wird, so dass sich über eine Amidbindung oberflächengebundene Polyacrylsäure ergibt (Journal of Chromatography, 358 (1986) 107-117). Das Verfahren hat den Vorteil, dass kein Kupplungsreagens benötigt wird. Als Teilschritt in der Herstellung des erfindungsgemäßen Reagens ist dieses Verfahren ebenfalls geeignet, solange die Voraussetzung erfüllt ist, dass die Bindung der Polyacrylsäure über möglichst wenige Bindungspunkte erfolgt.

Im kleineren Maßstab hat es sich als vorteilhafter erwiesen, die Amidbindung der Polyacrylsäure an die Trägeroberfläche mit Hilfe eines Kupplungsreagenzes zu erzeugen.

Als Aktivierungsreagenzien haben sich Karbaminsäureester, insbesondere 1-Ethoxy-N-ethoxycarbonyldihydrochinolin (EEDQ) bewährt, weil es ungiftig ist und leicht aus dem Adsorbens ausgespült werden kann. Die Erfindung soll jedoch nicht auf dieses Verfahren eingeschränkt sein, da zahlreiche weitere Kopplungsreagentien, u.a. Carbodiimide, ggf in Kombination mit N-Hydroxysuccinimid, eingesetzt werden können.

Das Adsorbens, erhältlich aus dem oben beschriebenen Verfahren, zeichnet sich dadurch aus, dass es
- keine Gerinnsel oder adhärierende Zellen nach der Passage von Blut aufweist.
- eine hohe Bindungskapazität, Bindungsgeschwindigkeit und Bindungsspezifität gegenüber arteriosklerosefördernden Lipoproteinen aufweist.
- durch Spülen mit polyanionhaltigen, isotonen Kochsalzlösungen oder polyanionfreien, hypertonen Kochsalzlösungen in Kombination mit physiologischen Spüllösungen vollständig regeneriert werden kann.
- dampfsterilisierbar und stabil ist.
- nicht zur Bildung von Abrieb oder Kleinteilen neigt und im Vergleich zu Hartgelen eine höhere Verformungs- und Bruchstabilität aufweist.
- stabil ist und keinen nachweisbaren Verlust von Polyacrylsäure aufweist.

Das oben beschriebenen Adsorbens ist verwendbar bei der extrakorporalen Adsorption von atherogenen Lipoproteinen oder anderen Blutbestandteilen oder Fremdstoffen aus Blut oder Plasma in einem Einwegsystem oder in einem individuellen Mehrwegsystem.

Neben der Verwendung mit Blut kann das Adsorbens auch an Stelle von anti-Apo-B-100-Sepharose bei der LDL-Apherese mit Plasma nach Abtrennung der Blutzellen, z.B. mit einem Zellseparator, eingesetzt werden. Dabei erfolgt die Verwendung kleiner Säulen, die während einer Behandlung mehrfach beladen und eluiert werden. Der Vorteil dieses sogenannten repetitiv-cyklischen Systems liegt in der theoretisch unbegrenzten Eliminationskapazität. Das erfindungsgemäßen Adsorbens ist für diese Form der Anwendung besonders vorteilhaft, weil es im Vergleich zu Sepharose schneller beladen, eluiert und gespült werden kann und zudem eine höhere Bindungskapazität aufweist.

Andererseits können Anwendungen, wie beispielsweise die bekannte Elimination von Lipopolysacchariden und Tumornekrosefaktor ohne vorherige Abtrennung der Blutzellen erfolgen. Weitere Anwendungsmöglichkeiten ergeben sich daraus, dass Liganden, z.B. Antikörper, zur Bindung von unerwünschten Biomolekülen oder Fremdstoffen leicht mittels Amidbindung zusätzlich an das Adsorbens gekuppelt werden können. Ein solcher zusätzlicher Reaktionsschritt kann auch unmittelbar vor der Anwendung erfolgen, wenn eine sterile Lösung des Liganden verfügbar ist.

Die vorliegende Erfindung liefert somit ein blutkompatibles, mehrfach regenerierbares Adsorptionsmaterial, insbesondere für arteriosklerosefördernde Lipoproteine. Dabei können die für die Säulenchromatographie bekannten Vorteile anorganischer Trägermaterialien, nämlich die starre Konsistenz und eine große, schnell und voll verfügbare Oberfläche für eine Anwendung mit Blut und Plasma in extrakorporaler Perfusion genutzt werden, weil die für eine Blutperfusion nachteiligen Eigenschaften dieser Materialien durch das erfindungsgemäße Herstellungsverfahren vollständig ausgeschaltet werden.

Die nachfolgenden Beispiele sollen die Erfindung näher erläutern, ohne sie jedoch darauf zu beschränken.

### Beispiele

### Beispiel 1

### Herstellung eines blutkompatiblen CPG-Polyacrylsäure-Derivates zur LDL-Eliminationsbehandlung

### a) Herstellung der 1,2-Dihydroxypropyl-3-oxypropylsilan/ 3-Aminopropyltriethoxysilan-Lösung

5 ml 3-Glycidoxypropyltrimethoxysilan (Nr. 50040, Fluka, Deisenhofen) werden mit 45 ml 0,1 M Schwefelsäure versetzt, für 50 Minuten bei 121°C im Autoklaven erhitzt, anschließend mit 1 M Natronlauge auf pH 3,5 eingestellt und mit destilliertem Wasser auf 250 ml aufgefüllt (2%ige Lösung). Zu dieser Lösung werden 125 µl 3-Aminopropyltriethoxysilan (Nr. 44,014-0, Sigma-Aldrich Chemie, Steinheim) gegeben, so dass es als 0,05% ige Lösung vorliegt (=40 Teile Diolsilan zu 1 Teil Aminosilan); ggf. wird der pH-Wert erneut auf 3,5 justiert.

### b) Silanisierung

Poröses Glas mit einer Partikelgröße von 130 - 200 µm, einer Porengröße von 1500 Å, einem Porenvolumen von 1260 mm³/g, und einer Oberfläche von 33 m²/g (z.B. Trisopor® Porous glass beads, Fa. Schuller, Steinach, Germany) wird auf einem 100 µm-Edelstahl-Prüfsieb durch Trockensiebung gründlich von Kleinteilen gereinigt, 4 Stunden in 10%iger Salpetersäure gekocht, mit Leitungswasser und destilliertem Wasser neutral gewaschen und 3 Stunden bei 110°C getrocknet. 50g des so vorbehandelten Glases werden in einem teflonummantelten, druckfest verschließbarem Gefäß mit 250 ml der hergestellten Silanlösung versetzt und für 15 Minuten im siedenden Wasserbad rotiert (Heidolph-Rührer, Typ RZR 1, niedrigste Stufe). Anschließend wird das Gefäß druckdicht verschlossen für 5 Stunden unter langsamer Rotation bei gleicher Temperatur belassen. Darauf folgt Spülung des silanisierten Glases unter filtriertem fließendem Leitungswasser auf einem 100 µm-Edelstahl-Prüfsieb für ca 5 Minuten, gefolgt von Dekantieren aus einem 2-1-Standzylinder mit destilliertem Wasser und 3-maligem Waschen mit je 150 ml Aceton (Nr. 27,072-5, Fa. Sigma, Deisenhofen). Schließlich wird das restliche Aceton in einem auf 120°C vorgeheizten, abgeschalteten Ofen abgedampft und das silanisierte Trägermaterial danach für 90 Minuten bei 110°C getrocknet. Gebundene Aminogruppen zeigen eine schwach positive Ninhydrinreaktion. Das Ergebnis des Silanol-Nachweises ist aus Tabelle 2 ersichtlich.

### c) Polyacrylsäurekopplung

10 ml 25%ige Polyacrylsäurelösung (Molekulargewicht 90 000, 25 Gew.% -Lösung in Wasser, Nr. 03326, Fa. Polysciences Europe GmbH, Eppelheim) werden mit 20 ml destilliertem Wasser verdünnt und der pH-Wert mit 1 M Natronlauge auf 4,5 eingestellt. Anschließend erfolgt unter Rühren die Zugabe von 20 ml Aceton. Danach wird die Polyacrylsäure-Lösung zu einer Lösung von 3 g 1-Ethoxy-N-ethoxycarbonyldihydrochinolin (Nr. 02541, Fluka, Deisenhofen) in 5 ml Aceton versetzt und durchmischt. Nach 5 Minuten erfolgt unter Rühren die tropfenweise Zugabe von ca. 5 ml destilliertem Wasser, bis eine klare Lösung entsteht. Diese wird 15 Minuten bei Raumtermperatur langsam gerührt, unter Rühren mit 100 ml destilliertem Wasser versetzt, auf das feuchte, gemäß der obigen Vorschrift vorbehandelte Glas gegeben und schnell durchmischt. Intensives Rühren oder Schütteln ist zu vermeiden. Nach 15-minütiger Kopplungszeit wird das Lösungsmittel abgesaugt, dreimal mit 1 M NaCI-Lösung gewaschen, in 1 M NaCl-Lösung für einige Minuten auf 60°C erhitzt, zehnmal mit Leitungswasser gewaschen, 10 Minuten in 1 M NaCl-Lösung zum Sieden erhitzt und dreimal mit destilliertem Wasser gewaschen. Anschließend wird das mit Polyacrylsäure (PAA)-beschichtete poröse Glas in destilliertem Wasser aufgenommen und in einem druckfest verschlossenen, teflonummantelten Behälter 60 Minuten auf 100°C erhitzt. Alternativ kann ein Heißdampfverfahren zur Sterilisation erfolgen.

### Beispiel 2

### In vitro-Perfusion von humanem Venenblut

Eine Kunststoffsäule mit einem Innendurchmesser von 9 mm und einer Höhe von 55 mm wurde bis zu einer Höhe von 31 mm (ca. 2 ml Adsorbens) mit dem gemäß Beispiel 1 hergestellten, mit Polyacrylsäure beschichtetem porösen Glasgranulat (Komgrößenbereich: 130 bis 200 µm, mittlere Porengröße: 1490 Å, Porenvolumen: 1260 mm³/g, Oberfläche 32,7 m²/g) luftblasenfrei befüllt und 20 ml frisch entnommenes, 10 Vol.% ACD-Stabilisator enthaltendes, menschliches Venenblut in Fraktionen aufgegeben. Die Perfusionsdauer betrug 45 Minuten, der Perfusionsdruck 85 mm Blut (gemessen vom Blutspiegel über dem Trägermaterial bis zum Auslauf). Nach der Blutperfusion wurde die Säule mit isotonischer Kochsalzlösung von Blut freigespült und zeigte danach keinerlei Rotfärbung als Hinweis auf eine Erythrozytenretention.
Die intensive Gelbfärbung des Säulenmaterials ist durch an Polyacrylsäure gebundene Lipoproteine bedingt. Nach spezifischer Elution mit 0,5% iger (w/v) Lösung von Polyacrylsäure-Na-Salz; Molekulargewicht 5000, in isotonischer Kochsalzlösung wird das Säulenmaterial wieder völlig weiß. Eine Analyse der Blutzusammensetzung vor bzw. nach der Perfusion ist in Tabelle 1 dargestellt.

### Vergleichsbeispiele

### Beispiel 3

Das in Beispiel 1 beschriebene unbehandelte Glas (Trisvpor® , Fa. Schuller) wurde gemäß DE 36 17 672 C2 epoxidiert und mit Lysin aminiert. Die Kupplung der Polyacrylsäure erfolgte analog zu Schritt c) in Beispiel 1. Die in vitro-Perfusion erfolgte gemäß Beispiel 2.
Eine Analyse der Blutzusammensetzung vor bzw. nach der Perfusion ist in Tabelle 1 dargestellt. Das Ergebnis des Silanol-Nachweises vor der Bindung der Polyacrylsäure ist aus Tabelle 2 ersichtlich.

### Beispiel 4

Das in Beispiel 1 beschriebene unbehandelte Glas (Trisopor®, Fa. Schuller) wurde gemäß EP 0143 369 mit einer 20%igen Lösung von 3-Glycidoxypropyltrimethoxysilan in Aceton bei 50°C in einem Zeitraum von 40 Stunden epoxidiert. Aminierung und Kopplung der Polyacrylsäure erfolgten gemäß Schritt c) in Beispiel 1. Die in vitro-Perfusion erfolgte gemäß Beispiel 2. Eine Analyse der Blutzusammensetzung vor bzw. nach der Perfusion ist in Tabelle 1 dargestellt. Das Ergebnis des Silanol-Nachweises vor der Bindung der Polyacrylsäure ist aus Tabelle 2 ersichtlich.

### Beispiel 5

Das Herstellungsverfahren erfolgte analog zu dem in Beispiel 1 beschriebenen, mit dem einzigen Unterschied, dass die Herstellung der Silanlösung ohne Verwendung von Aminosilan erfolgte. Eine Analyse der Blutzusammensetzung vor bzw. nach der Perfusion ist in Tabelle 1 dargestellt. Das Ergebnis des Silanol-Nachweises ist aus Tabelle 2 ersichtlich.

**Tabelle 1**

| Analyse der Blutzusammensetzung vor bzw. nach der Perfusion | | | | | |
|---|---|---|---|---|---|
| Parameter | Vor Säulen-Passage | Beispiel 2 Diolsilan¹/Aminopropylsilan²-PAA | Beispiel 3 Epoxysilan³- Lysinspacer- PAA | Beispiel 4 Epoxysilan³ in Aceton -NH₂-PAA | Beispiel 5 in Diolsilan¹- PAA⁴, kein Aminopropylsilan² |
| Cholesterin mg/dl | 184 | 71 | 68 | 67 | 180 |
| LDL-Cholesterin | 130 | 20 | 20 | 23 | 128 |
| HDL-Cholesterin | 41 | 43 | 39 | 36 | 39 |
| Triglyceride | 86 | 56 | 56 | 58 | 84 |
| LDH U/l | 154 | 156 | 157 | 155 | 146 |
| | | | | | |
| Leukozyten x10³/µl | 3,5 | 2,8 | 2,6 | 2,6 | 2,6 |
| | | | | | |
| Thrombozyt. x10³/µl | 158 | 148 | 156 | 99 | 156 |
| Hb-Konzentr. g/dl | 11,9 | 12,2 | 12,3 | 12,2 | 12,3 |
| Visueller Nachweis von Erythrozyten nach Spülung⁵ | | nein | ja | ja | ja |
| Eluierbarkeit von LDL mit Polyacrylsäure | | vollständig | Teilweise | teilweise | - |

| | | | | | |
|---|---|---|---|---|---|
| ¹ 1,2 Dihydroxypropyl-3-oxypropylsilan | | | | | |
| ² Aminopropyltriethoxysilan | | | | | |
| ³ 3-Glycidoxypropyltrimethoxisilan | | | | | |
| ⁴ Wegen des Fehlens von Aminogruppen ist keine Polyacrylsäure gebunden | | | | | |
| ⁵ mit isotonischer Kochsalzlösung | | | | | |

Alle Polyacrylsäure-beschichteten Adsorbentien binden aus einem Blutvolumen entsprechend dem 10-fachen Volumen des Adsorptionsmaterials über 80% des LDL-Cholesterins, während Triglyceride in geringem Maße, HDL-Cholesterin dagegen gar nicht gebunden wird. Bemerkenswert ist insbesondere, dass das Adsorptionsmaterial aus Beispiel 5 gar keine Lipoproteine bindet. Das erfindungsgemäße Adsorbens unterscheidet sich somit grundlegend von dem in DE 40 18 778 A1 beschriebenen. Tabelle 1 ist zudem zu entnehmen, dass die an unbeschichtetem Glas in hohem Ausmaß erfolgende Thrombozytenretention bereits vollständig durch die erfindungsgemäße Diolbeschichtung alleine aufgehoben wird. Auffällig ist, dass einzig das gemäß Beispiel 4 hergestellte Adsorptionsmaterial Thrombozyten in höherem Ausmaß retiniert und schon deshalb für eine Anwendung mit Blut ungeeignet ist.

Wie bereits dargestellt, ist ein weiteres Beurteilungskriterium für die Blutkompatibilität der Umfang anhaftender, nicht mit isotonischer Kochsalzlösung ausspülbarer Erythrozyten. Hier wird die Überlegenheit des erfindungsgemäßen Adsorbens bzw. Beschichtungsverfahrens deutlich, welches als einziges in der Lage ist, so-wohl die Thrombozyten- als auch die Erythrozytenretention vollständig aufzuheben.

Ebenso erfolgt eine vollständige Eluierung von LDL mit Polyacrylsäure einzig bei dem Adsorptionsmaterial der vorliegenden Erfindung. Während das Adsorbens aus Beispiel 5 LDL gar nicht erst adsorbiert, findet die Eluierung in den Trägermaterialien der anderen Beispiele nur teilweise statt.

**Tabelle 2**

| Anfärbung des trockenen Glases mit 0,05% Methylrot in Toluol bei verschiedenen Beschichtungen | | | | |
|---|---|---|---|---|
| Unbeschichtet | Beispiel 2 Diolsilan¹/ Aminopropylsilan²⁻ | Beispiel 3 (Vergleich) Epoxysilan³- Lysinspacer | Beispiel 4 (Vergleich) Epoxysilan³ in A-ceton -NH₂ | Beispiel 5 (Vergleich) Diolsilan¹ |
| ++ | - | + | + | - |

| | | | | |
|---|---|---|---|---|
| ¹1,2 Dihydroxypropyl-3-oxypropylsilan | | | | |
| ² Aminopropyltriethoxysilan | | | | |
| ³ 3-Glycidoxypropyltrimethoxisilan | | | | |

Bei dem in Tabelle 2 zum Einsatz kommenden Verfahren handelt es sich, wie in der Beschreibung bereits erwähnt, um einen empfindlichen Nachweis freier Silanolgruppen, die den gelben Farbstoff adsorbieren und durch einen Farbumschlag zu einer Rotfärbung der Trägeroberfläche führen. Die Reaktion ist bereits bei der erfindungsgemäßen Beschichtung nur mit 1,2-Dihydroxypropyl-3-oxypropylsilan vollständig negativ. Im Vergleich der Beispiele 2 und 5 wird jedoch deutlich, dass es zusätzlich der Polyacrylsäure bedarf, um eine Retention von Erythrozyten zu verhindern.

## Patentansprüche

1. Adsorbens zur Perfusion von Blut oder Plasma, bestehend aus einem festen, siliciumhaltigen, anorganischen, porösen Trägermaterial, an das zumindest ein hydrophiles und ein kupplungsfähiges Organosilan gebunden sind, wobei das hydrophile Organosilan ein Diolsilan und das kupplungsfähige Organosilan ein Aminosilan ist, **dadurch gekennzeichnet, dass**
a) ein Polyanion kovalent an das Aminosilan gebunden ist, wobei das Polyanion ein Acrylpolymer ist,
b) keine freien Silanolgruppen auf der Trägeroberfläche mehr vorhanden sind und
c) der Anteil des Aminosilans relativ zum Diolsilan 0,1-20% beträgt.

2. Adsorbens nach Anspruch 1, **dadurch gekennzeichnet, dass** das Trägermaterial poröses Glas oder Kieselgel ist.

3. Adsorbens nach Ansprüchen 1 und 2, **dadurch gekennzeichnet, dass** das Trägermaterial sphärische oder nicht-sphärische Partikel mit einem Durchmesser von 50 - 250 µm enthält.

4. Adsorbens nach den Ansprüchen 1 - 3, **dadurch gekennzeichnet, dass** das Trägermaterial sphärische oder nicht-sphärische Partikel mit einem Durchmesser von 100 - 200 µm enthält.

5. Adsorbens nach den Ansprüchen 1 - 4, **dadurch gekennzeichnet, dass** das Trägermaterial Poren mit einem mittleren Durchmesser von 50 nm - 300 nm (500 Å -3000 Å) aufweist.

6. Adsorbens nach Anspruch 1, **dadurch gekennzeichnet, dass** das Aminosilan 3-Aminopropyltriethoxysilan ist.

7. Adsorbens nach Anspruch 1, **dadurch gekennzeichnet, dass** das Acrylpolymer ein Polymer oder Copolymer der Acrylsäure, Methacrylsäure oder Hydroxyethylmethacrylsäure ist.

8. Adsorbens nach einem der Ansprüche 1 und 7, **dadurch gekennzeichnet, dass** das Acrylpolymer Polyacrylsäure mit einem mittleren Molekulargewicht von 5 x 10³ - 5 x 10⁶ D ist.

9. Adsorbens nach Anspruch 8, **dadurch gekennzeichnet, dass** die Polyacrylsäure ein mittleres Molekulargewicht von 5 x 10⁴ - 5 x 10⁵ D aufweist.

10. Adsorbens nach Anspruch 1, **dadurch gekennzeichnet, dass** das Diolsilan 1,2-Dihydroxypropyl-3-oxypropylsilan ist.

11. Adsorbens nach den Ansprüchen 1 - 10, verwendbar bei der extrakorporalen Adsorption von Lipoproteinen geringer Dichte, anderen Blutbestandteilen oder Fremdstoffen aus Blut oder Plasma mittels Perfusion in einem Einwegsystem oder in einem individuellen Mehrwegsystem.

12. Verfahren zur Herstellung eines Adsorbens nach einem der Ansprüche 1 - 11, das folgende Schritte beinhaltet:
a) Bindung eines Aminosilans und eines Diolsilans an die Trägeroberfläche unter vollständiger Umsetzung der freien Silanolgruppen auf der Trägeroberfläche
b) Kupplung eines Acrylpolymers in einem pH-Wertbereich von 1,5 - 8,5.

13. Verfahren gemäß Anspruch 12, wobei das Diolsilan vor der Bindung auf die Oberfläche durch Hydrolyse eines Epoxysilans gewonnen wird.

14. Verfahren gemäß Anspruch 13, wobei das Epoxysilan 3-Glycidoxypropyltrimethoxysilan ist.

## Claims

1. Adsorbents for blood or plasma perfusion, composed of a solid, silicon-containing, inorganic, porous supporting material, to which at least one hydrophilic and one coupling organosilane are bonded, whereby the hydrophilic organosilane is a diol silane and the coupling organosilane is an aminosilane, **characterised by** the fact that
a) a polyanion is covalently bonded to the aminosilane, whereby the polyanion is an acrylic polymer,
b) there are no longer any free silanol groups on the surface of the supporting material and
c) the proportion of aminosilane relative to the diol silane amounts to 0.1-20%.

2. Adsorbents in accordance with Claim 1, **characterised by** the fact that the supporting material is porous glass or silica gel.

3. Adsorbents in accordance with Claims 1 and 2, **characterised by** the fact that the supporting material contains spherical or non-spherical particles with a diameter of 50-250 µm.

4. Adsorbents in accordance with Claims 1 - 3, **characterised by** the fact that the supporting material contains spherical or non-spherical particles with a diameter of 100 -200 µm.

5. Adsorbents in accordance with Claims 1 - 4, **characterised by** the fact that the supporting material has pores with an average diameter of 50 nm - 300 nm (500 Å -3000 Å).

6. Adsorbents in accordance with Claim 1, **characterised by** the fact that the aminosilane is 3-aminopropyltriethoxysilane.

7. Adsorbents in accordance with Claim 1, **characterised by** the fact that the acrylic polymer is a polymer or copolymer of acrylic acid, methacrylic acid or hydroxyethyl methacrylic acid.

8. Adsorbents in accordance with one of the Claims 1 and 7, **characterised by** the fact that the acrylic polymer is polyacrylic acid with an average molecular weight of 5 x 10³ - 5 x 10⁶ d.

9. Adsorbents in accordance with Claim 8, **characterised by** the fact that the polyacrylic acid has an average molecular weight of 5 x 10⁴ - 5 x 10⁵ d.

10. Adsorbents in accordance with Claim 1, **characterised by** the fact that the diol silane is 1,2-dihydroxypropyl-3-oxypropylsilane.

11. Adsorbents in accordance with Claims 1 - 10, which can be used with the extracorporeal adsorption of low density lipoproteins, other blood constituents or foreign materials from blood or plasma by means of perfusion in a single-use system or in an individual, reusable system.

12. Procedure for producing an adsorbent in accordance with one of the Claims 1 - 11, which contains the following steps
a) bonding an aminosilane and a diol silane on the surface of the supporting material with the complete conversion of the free silanol groups on the surface of the supporting material
b) coupling an acrylic polymer within a pH-value range of 1.5 - 8.5.

13. Procedure in accordance with Claim 12, whereby the diol silane is obtained before binding to the surface by means of the hydrolysis of an epoxysilane.

14. Procedure in accordance with Claim 13, whereby the epoxysilane is 3-glycidoxypropyltrimethoxysilane.

## Revendications

1. Absorbant pour perfusion de sang ou de plasma, constitué d'un substrat solide, à teneur en silicium, inorganique et poreux, auquel sont liés au moins un organosilane hydrophile et un organosilane apte au couplage, l'organosilane hydrophile étant un silane diol et l'organosilane apte au couplage étant un silane aminé, et **caractérisé par** le fait
a) Qu'un polyanion covalent est lié au silane aminé, et que le polyanion est un polymère acrylique,
b) Que la surface du substrat ne comporte plus aucun groupe libre de silanol et
c) Que la proportion de silane aminé par rapport au silane diol est de 0,1-20%.

2. Absorbant selon la revendication 1, **caractérisé par le fait que** le substrat est du verre poreux ou du gel de silice.

3. Absorbant selon les revendications 1 et 2, **caractérisé par le fait que** le substrat contient des particules sphériques ou non sphériques d'un diamètre de 50 - 250 µm.

4. Absorbant selon les revendications 1 - 3, **caractérisé par le fait que** le substrat contient des particules sphériques ou non sphériques d'un diamètre de 100 - 200 µm.

5. Absorbant selon les revendications 1 - 4, **caractérisé par le fait que** le substrat présente des pores d'un diamètre moyen de 50 nm - 300 nm (500 Å -3000 Å).

6. Absorbant selon la revendication 1, **caractérisé par le fait que** le silane aminé est un 3 aminopropyl triéthoxy-silane.

7. Absorbant selon la revendication 1, **caractérisé par le fait que** le polymère acrylique est un polymère ou un copolymère d'acrylates, de méthacrylates ou de méthacrylates d'hydroxyéthyle.

8. Absorbant selon l'une des revendications 1 et 7, **caractérisé par le fait que** le polymère acrylique est un polyacrylate affichant un poids moléculaire moyen de 5 x 10³ - 5 x 10⁶ D.

9. Absorbant selon la revendication 8, **caractérisé par le fait que** le polyacrylate présente un poids moléculaire moyen de 5 x 10⁴ - 5 x 10⁵ D.

10. Absorbant selon la revendication 1, **caractérisé par le fait que** le silane diol est un silane 1,2 dihydroxypropyl 3 oxypropylène.

11. Absorbant selon les revendications 1 - 10, utilisable pour l'adsorption extra-corporelle de lipoprotéines de faible densité, d'autres constituants sanguins ou de substances étrangères de sang ou de plasma, à l'aide d'une perfusion dans un système jetable ou un système individuel à utilisation multiple.

12. Procédé de fabrication d'un absorbant selon l'une des revendications 1 - 11, comprenant les étapes suivantes :
a) Liaison d'un silane aminé et d'un silane diol à la surface du substrat sous mutation complète de groupes libres de silanole sur la surface du substrat
b) Accouplement d'un polymère acrylique dans une plage de valeur pH se situant entre 1,5 et 8,5.

13. Procédé selon la revendication 12, dans lequel le silane diol est obtenu avant la liaison sur la surface par hydrolyse d'un époxy silane.

14. Procédé selon la revendication 12, dans lequel l'époxy silane est un silane trimethoxyle 3-glycidoxypropyle.
